# EUROPEAN PATENT APPLICATION

(11) **EP 1 864 663 A1**
(43) Date of publication of application: **12.12.2007**
(21) Application number: 06730311.5
(22) Date of filing: 28.03.2006
(51) Int. Cl.: A61K 31/50, A61K 31/192, A61K 31/415, A61K 31/426, A61P 29/00, A61P 43/00, C07D 237/14

(54) **PREVENTIVE AND/OR THERAPEUTIC AGENT FOR RHEUMATOID ARTHRITIS**

(30) Priority: 29.03.2005 US 665852 P; 30.11.2005 US 740658 P; 27.12.2005 US 756254 P; 13.01.2006 US 758569 P
(71) Applicant: Kowa Co., Ltd., Nagoya-shi, Aichi 460-8625 (JP)
(72) Inventor: TABUNOKI, Yuichiro, Tokyo, 203-0051 (JP); KOSHI, Tomoyuki, Saitama, 353-0006 (JP)
(74) Representative: Blodig, Wolfgang
(86) International application number: PCT/JP2006/306363
(87) International publication number: WO 2006/104172

(57) **Abstract**

The present invention relates to a preventive and/or therapeutic agent for rheumatoid arthritis. The present invention provides a preventive and/or therapeutic agent for rheumatoid arthritis containing
2-benzyl-5-(4-chlorophenyl)-6-[4-(methylthio)phenyl]-2H-pyr idazin-3-one and a COX2 inhibitor.

## Description

### Technical Field

The present invention relates to a preventive and/or therapeutic agent for rheumatoid arthritis.

### Background Art

Rheumatoid arthritis is a disease which involves inflammation accompanied with swelling and pain in many joints, and the progression of symptoms over a long period of time leads to irreversible deformation in the joints and functional disorders, which causes a significant deterioration in the quality of life (QOL) of the patient. In Japan, 0.6% of the total population, and 1% of the population over age 30 suffer from rheumatoid arthritis, and particularly in recent years, along with the progress of aging society, there is observed a tendency for an increase in the number of elderly rheumatoid patients.

Disease stages of rheumatoid arthritis can be divided into the following four stages: (1) initial stage: a stage in which joint pain and arthritis are observed, but definite diagnosis of rheumatoid arthritis still cannot be made; (2) early stage: a stage in which definite diagnosis of rheumatoid arthritis can be made, but still there is no or little irreversible change (early stage of rheumatoid arthritis generally refers to the first 1 to 2 years from the onset of the disease); (3) progressive stage: a stage in which irreversible changes appear, and strong systemic symptoms such as fatigue, mild fever and weight loss emerge; and (4) late stage: a stage in which inflammation in the joints is almost going to remission, but irreversible changes such as deformation and contracture remain strong, with the predominant symptoms being pain and functional disorders. The therapeutic method varies with the respective disease stages. There have been reported from some studies that the onset of rheumatoid arthritis is associated with genetic factors or acquired factors (i.e. , being an infectious disease) ; however, the cause of the disease is still unknown, and it is still impossible for the present to achieve complete cure and prevention. The goal of treatment under the present circumstances lies in trying to enhance the QOL of patients from physical, mental and social aspects, by making early diagnosis of rheumatoid arthritis, suppressing inflammation caused by rheumatoid arthritis as rapidly as possible to the maximum extent, and thereby preventing the emergence of irreversible changes or inhibiting progress thereof. Therefore, upon the treatment of the disease, the patients are given sufficient explanation on the disease or the therapeutic methods for the disease, and then various means of treatment such as physical therapy, kinesitherapy, drug therapy and surgical therapy are applied to the patients.

In drug therapy, non-steroidal antiinflammatory drugs (NSAIDs), disease-modifying antirheumatic drugs (DMARDs) and steroid drugs are being used in the clinical field. Recently, biological agents such as antibodies targeted against proinflammatory cytokines are also used (see Non-Patent Document 1).

The term NSAIDs is used as a synonym for cyclooxygenase (COX) inhibitors, and most of the drugs belonging to this category have a COX inhibitory action. COX is known to have two isotypes such as COX1 and COX2 at the gene level. Furthermore, COX3 has been recently discovered as a variant of COX1 (see Non-Patent Document 2). The object of prescribing a COX inhibitor to the patients of rheumatoid arthritis is mainly related to manifestation of immediately effective analgesic action, and COX inhibitors are hardly expected to have an effect of remitting rheumatoid arthritis per se. However, since DMARDs that are used to actually remit rheumatoid arthritis are slow in exhibiting the effect, generally COX inhibitors are used in combination therewith for the purpose of improving the QOL until the effect of DMARDs appears. Under such circumstances, COX inhibitors are being prescribed to many rheumatoid arthritis patients, but with those classical COX inhibitors having an inhibitory effect against both COX1 and COX2 (for example, indomethacin, aspirin), there is a problem of the onset of serious gastrointestinal disorder in the stomach. In this regard, COX2-selective inhibitors with reduced gastrointestinal disorder have been developed in recent years (see Non-Patent Document 3). Also, it has been recently confirmed that COX2-selective inhibitors have serious side effects of raising the risk of onset of cardiovascular diseases (see Non-Patent Document 4), and the present situation is such that the use of high dosage or long term use of COX inhibitors must be restricted in some ways.

Interleukin 1β (IL-1β), which is a proinflammatory cytokine, is recognized with stimulation of production thereof in many diseases such as, for example, rheumatoid arthritis, arthrosis deformans, osteoporosis, inflammatory bowel disease, immune deficiency syndrome, septicemia, hepatitis, nephritis, ischemic diseases, insulin-dependent diabetes, arteriosclerosis, Parkinson's disease, Alzheimer's disease, and leukemia, and is known to induce synthesis of enzymes which are conceived to be associated with inflammation, such as collagenase, COX and PLA2. IL-1β is also known to cause articular damage which is very similar to rheumatoid arthritis when intra-articularly injected to animals. Thus, IL-1β inhibitors are studied and developed as a drug for treating inflammatory diseases, and materials derived from biological substances such as IL-1 receptor antagonist (see Non-Patent Document 5), low molecular weight compounds such as T-614 (see Non-Patent Document 6), S-2474 (see Non-Patent Document 7), 2-benzyl-5-(4-chlorophenyl)-6-[4-(methylthio)phenyl]-2H-pyr idazin-3-one (see Patent Document 1), and FR133605 (see Non-Patent Document 8) are known.

Pathological conditions of rheumatoid arthritis vary considerably among patients in terms of age, disease stage, complications, side effects, QOL, and the like. Also, there exists no ultimate therapeutic drug, and even a phenomenon called "escape phenomenon" is observed, in which a therapeutic drug having a well established controlling effect on symptoms suddenly begins to lose the effect. Under such circumstances, when a drug therapy is to be performed, change of drugs or combined use of drugs is frequently employed, and thus clinical studies on the method of use of such drugs are also being actively carried out.

Although there are reports on drugs having both the COX2 inhibitory action and the IL-1β inhibitory action (see Patent Document 2 and Non-Patent Documents 9 and 10), nothing is known about the effect, particularly the effect on rheumatoid arthritis, of combined use of a drug having the COX2 inhibitory action and an IL-1β inhibitor.
[Patent Document 1] International Patent Application Publication No. WO 99/25697
[Patent Document 2] International Patent Application Publication No. WO 03/084936
[Non-Patent Document 1] American College of Rheumatology Subcommittee on Rheumatoid Arthritis Guidelines, Arthritis & Rheumatism 46, pp 328-346, 2002
[Non-Patent Document 2] Proc. Natl. Acad. Sci. USA. 99, pp. 13926-13931, 2002
[Non-Patent Document 3] Proc. Natl. Acad. Sci. USA. 96, pp. 7563-7568, 1999
[Non-Patent Document 4] N. Engl. J. Med. 351, pp. 1707-1711, 2004, Circulation 111, pp. 249, 2005
[Non-Patent Document 5] Arthritis & Rheumatism 42, pp 498-506, 1999
[Non-Patent Document 6] J. Pharmacobio-Dyn. 11, pp 649-655, 1992
[Non-Patent Document 7] YAKUGAKU ZASSHI 123, pp 323-330, 2003
[Non-Patent Document 8] J. Rheumatol. 23, pp 1778-1783, 1996
[Non-Patent Document 9] Jpn. J. Pharmacol. 67, pp. 305-314, 1995
[Non-Patent Document 10] J. Pharmacobio-Dyn. 15, pp. 649-655, 1992

### Disclosure of the Invention

### Problems to be Solved by the Invention

It is an object of the present invention to provide a drug having an excellent effect on the treatment and prevention of rheumatoid arthritis.

### Means for Solving the problems

Under such circumstances as described above, the inventors of the present invention have devotedly conducted research, and as a result, found that combined use of 2-benzyl-5-(4-chlorophenyl)-6-[4-(methylthio)phenyl]-2H-pyr idazin-3-one, which is an IL-1β inhibitor, and a drug having a COX2 inhibitory action (COX2 inhibitor), results in an excellent effect of suppressing arthritis. The present invention has been accomplished on the basis of this finding.

Thus, the present invention is to provide a preventive and/or therapeutic agent for rheumatoid arthritis comprising 2-benzyl-5-(4-chlorophenyl)-6-[4-(methylthio)phenyl]-2H-pyr idazin-3-one and a COX2 inhibitor.
The present invention is to provide a method of treating rheumatoid arthritis, the method comprising administering an effective amount of 2-benzyl-5-(4-chlorophenyl)-6-[4-(methylthio)phenyl]-2H-pyr idazin-3-one and a COX2 inhibitor.
Furthermore, the present invention is to provide use of 2-benzyl-5-(4-chlorophenyl)-6-[4-(methylthio)phenyl]-2H-pyr idazin-3-one and a COX2 inhibitor for the production of a preventive and/or therapeutic agent for rheumatoid arthritis.

### Effect of the Invention

The preventive and/or therapeutic agent for rheumatoid arthritis of the present invention can be administered orally, and exhibits an excellent suppressive action on arthritis with reduced side effects. Thus, the agent is useful for the prevention and/or treatment of rheumatoid arthritis.

### Brief Description of the Drawings

The drawings are all presented with average values and standard errors, and with the respective exemplary values.
Fig. 1 is a graph showing the Edema Index and inhibition rate in a rat collagen-induced arthritis model, in the case of combined administration of 2-benzyl-5-(4-chlorophenyl)-6-[4-(methylthio)phenyl]-2H-pyr idazin-3-one and diclofenac sodium;
Fig. 2 is a graph showing the Edema Index and inhibition rate in a rat collagen-induced arthritis model, in the case of combined administration of 2-benzyl-5-(4-chlorophenyl)-6-[4-(methylthio)phenyl]-2H-pyr idazin-3-one and ketoprofen;
Fig. 3 is a graph showing the Edema Index and inhibition rate in a rat collagen-induced arthritis model, in the case of combined administration of 2-benzyl-5-(4-chlorophenyl)-6-[4-(methylthio)phenyl]-2H-pyr idazin-3-one and meloxicam;
Fig. 4 is a graph showing the Edema Index and inhibition rate in a rat collagen-induced arthritis model, in the case of combined administration of 2-benzyl-5-(4-chlorophenyl)-6-[4-(methylthio)phenyl]-2H-pyr idazin-3-one and celecoxib; and
Fig. 5 is a graph showing the Edema Index and inhibition rate in a rat collagen-induced arthritis model, when the content of celecoxib in the preventive and/or therapeutic agent for rheumatoid arthritis of the present invention was changed.

### Best Mode for Carrying Out the Invention

2-Benzyl-5-(4-chlorophenyl)-6-[4-(methylthio)phenyl]-2H-pyridazin-3-one (hereinafter, may be sometimes referred to as Drug A), which is used in the preventive and/or therapeutic agent for rheumatoid arthritis of the present invention, is a known substance, and can be produced by, for example, the method described in International Patent Application Publication No. WO 99/25697, or a similar method. That is, p-chlorophenylacetic acid is reacted with thioanisole in the presence of a condensing agent such as polyphosphoric acid, to obtain 2-(4-chlorophenyl)-4'-(methylthio)acetophenone (compound a). The compound a is reacted with a base such as potassium t-butoxide in tetrahydrofuran, and then ethyl bromoacetate is added to the reaction system, to thereby obtain ethyl 2-(4-chlorophenyl)-4-[4-(methylthio)phenyl]-4-oxobutanoate (compound b). The compound b is reacted with hydrazine hydrate in ethanol, to obtain 5-(4-chlorophenyl)-6-[4-(methylthio)phenyl]-4,5-dihydro-2H-pyridazin-3-one (compound c). The compound c thus obtained is reacted with benzyl bromide in a solvent such as N,N-dimethylformamide, in the presence of a base such as potassium carbonate, and Drug A can be thus produced.

The COX2 inhibitor that can be used in the preventive and/or therapeutic agent for rheumatoid arthritis of the present invention may be exemplified by a COX2 inhibitor derived from a carboxylic acid, an enolic acid, a methanesulfonic acid, a tricyclic compound, or the like. Examples of the carboxylic acid-based COX2 inhibitors include COX2 inhibitors derived from salicylic acid, such as aspirin; from anthranilic acid or fenamic acid, such as mefenamic acid, flufenamic acid and tolfenamic acid; from phenylacetic acid, such as diclofenac or a salt thereof, alclofenac, fenbufen, nabumetone and lumiracoxib; from indoleacetic acid, such as indomethacin, sulindac, indomethacin farnesyl, proglumetacin maleate, acemetacin and amfenac; from heterocyclic acetic acid, such as tolmetin; from pyranoacetic acid, such as etodolac; and from propionic acid, such as ibuprofen, pranoprofen, naproxen, ketoprofen or a salt thereof, flurbiprofen, tiaprofen, pranoprofen, loxoprofen, oxaprozin, alminoprofen and zaltoprofen.
In addition, the salt of diclofenac and the salt of ketoprofen are not particularly limited as long as they are pharmacologically acceptable salts, and examples thereof include inorganic salts such as sodium salts and potassium salts, or organic salts.
Examples of the enolic acid-based COX2 inhibitor include COX inhibitors derived from pyrazolone, such as phenylbutazone, clofezone, febrazone and oxyphenylbutazone; and from oxicam, such as piroxicam, ampiroxicam, tenoxicam, lornoxicam and meloxicam. Examples of the methanesulfonic acid-based COX2 inhibitor include nimesulide, flosulide, T-614 and the like. Examples of the tricyclic-based COX2 inhibitors include rofecoxib, celecoxib, valdecoxib, etoricoxib and the like. In addition, if these compounds have optical activities, their optical isomers are also included. For the COX2 inhibitor, a COX2 inhibitor derived from a carboxylic acid, an enolic acid or a tricyclic compound is preferred, and a COX2 inhibitor derived from phenylacetic acid, propionic acid, oxicam or a tricyclic compound is more preferred, with diclofenac or a salt thereof, ketoprofen or a salt thereof, meloxicam or celecoxib (hereinafter, may be sometimes referred to as Drug B) being particularly preferred. With regard to these drugs, commercially available products, for example, products from Sigma-Aldrich Company for diclofenac and ketoprofen; products from Wako Pure Chemical Industries, Ltd. for meloxicam; and products from Desynth S.A. for celecoxib, can be used.

The contents of the Drug A and the COX2 inhibitor in the preventive and/or therapeutic agent for rheumatoid arthritis of the present invention are such that the mass ratio (Drug A:COX2 inhibitor) is from 1000:1 to 1:30. In the case of the carboxylic acid-based COX2 inhibitors, the ratio is more preferably from 300:1 to 1:3, and particularly preferably from 100:1 to 1:1. In the case of the enolic acid-based and tricyclic-based COX2 inhibitors, the ratio is particularly preferably from 10:1 to 3:10.

The dosage form for the preventive and/or therapeutic agent for rheumatoid arthritis of the present invention is not particularly limited, and can be appropriately selected in accordance with the purpose of treatment. Examples of the dosage form include orally administered forms such as tablets, capsules, granules, film-coated tablets, powders, and syrups, or parenterally administered forms such as injections, suppositories, inhalants, transdermal preparations, eye drops, and nasal drops. Orally administered forms are preferred.

For the preventive and/or therapeutic agent for rheumatoid arthritis of the present invention, separate pharmaceutical preparations respectively containing the active ingredients, that is, Drug A and a COX2 inhibitor, may be prepared, and they may be administered simultaneously or separately at an interval. Alternatively, it is also possible to administer a single pharmaceutical preparation containing both active ingredients together.

A pharmaceutical preparation suitable for such dosage forms may be appropriately combined with pharmacologically acceptable carriers, for example, excipients or bulking agents such as starches, lactose, sucrose, mannitol, and silicic acid; disintegrants such as agar, calcium carbonate, potato or tapioca starch, alginic acid, and silicate complexes; binders such as hydroxypropylmethylcellulose, methylcellulose, carboxymethylcellulose sodium, alginates, gelatin, polyvinylpyrrolidone, sucrose, and gum arabic; lubricants such as talc, calcium stearate, magnesium stearate, solid polyethylene glycols, sodium lauryl sulfate, and mixtures thereof; diluents such as lactose and corn starch; buffers of organic acids such as citric acid, phosphoric acid, tartaric acid and lactic acid, of inorganic acids such as hydrochloric acid, of alkali hydroxides such as sodium hydroxide and potassium hydroxide, and of amines such as triethanolamine, diethanolamine and diisopropanolamine; antiseptics such as paraoxybenzoic acid esters and benzalkonium chloride; emulsifying agents, such as anionic surfactants such as calcium stearate, magnesium stearate and sodium lauryl sulfate, cationic surfactants such as benzalkonium chloride, benzetonium chloride and cetylpyridinium chloride, and nonionic surfactants such as glyceryl monostearate, sucrose fatty acid esters, polyoxyethylene-hydrogenated castor oil, polyoxyethylene sorbitan fatty acid esters, polyoxyethylene fatty acid esters and polyoxyethylene alkyl ethers; and stabilizing agents such as sodium sulfite, sodium bisulfite, dibutylhydroxytoluene, butylhydroxyanisole, and EDTA. In addition, odor-suppressors, dispersants, preservatives, flavoring agents and the like may appropriately be combined and used, according to necessity.

According to the present invention, the dosage of 2-benzyl-5-(4-chlorophenyl)-6-[4-(methylthio)phenyl]-2H-pyr idazin-3-one is appropriately selected depending on the body weight, age, gender and symptoms of the patient, but typically for an adult, the compound may be administered in an amount of 2 to 320 mg, preferably 4 to 160 mg, per day. The dosage of the COX2 inhibitor may vary depending on the type of the inhibitor, but for an adult, the inhibitor may be administered in an amount of 1 to 1500 mg per day.
The dosage of a carboxylic acid-based or enolic acid-based COX2 inhibitor may vary depending on the type of the inhibitor, but for an adult, the inhibitor may be administered in an amount of 1 to 1500 mg per day. For example, in the case of using diclofenac, 25 to 300 mg, and preferably 100 to 150 mg, per day may be administered. In the case of using ketoprofen, 25 to 400 mg, and preferably 100 to 300 mg, per day may be administered. In the case of using meloxicam, 1 to 50 mg, and preferably 5 to 20 mg, per day may be administered.
The dosage of a tricyclic-based COX2 inhibitor may vary depending on the type of the inhibitor, but for an adult, the inhibitor may be administered in an amount of 10 to 1500 mg per day. For example, in the case of using celecoxib, 100 to 800 mg, and preferably 200 to 400 mg, per day may be administered.

### EXAMPLES

Hereinafter, the present invention will be described in more detail with reference to Examples, but the present invention is not intended to be limited to these Examples.

### EXAMPLE 1

With regard to combined administration of 2-benzyl-5-(4-chlorophenyl)-6-[4-(methylthio)phenyl]-2H-pyr idazin-3-one (Drug A) and diclofenac sodium (Drug B-1); combined administration of Drug A and ketoprofen (Drug B-2); combined administration of Drug A and meloxicam (Drug B-3); combined administration of Drug A and celecoxib (Drug B-4); and individual administration of the drugs, the edema suppressing effect in both hindlimbs was determined in a rat collagen-induced arthritis model as described below (FDA, CBER, CDER, CDRH: Guidance for industry - Clinical development programs for drugs, devices, and biological products for the treatment of rheumatoid arthritis (RA)-.(1999)).

Female Lewis rats (LEW/Crj) (obtained from Charles River Laboratories Japan, Inc.) were used as test animals.
For each 8-week-old LEW/Crj rat, the volume of a portion extending from the ankle to the paw was measured for both hindlimbs (hindlimbs volume) by means of a plethysmometer for small animals (TK-101CMP, manufactured by Unicom), and the total volume was taken as the hindlimbs volume upon initiation of test (Pre value). This Pre value was used as an index to divide the rats into groups by one-parameter-based block randomization, so that the values in the respective groups averaged out.

The collagen emulsion for sensitization used to induce arthritis in rats was prepared by homogenizing 0.3% Type II collagen liquid (manufactured by Collagen Research Center), Adjuvant Peptide (manufactured by Peptide Institute, Inc.) and Adjuvant Incomplete Freund (manufactured by DIFCO), using a Handy Micro Homogenizer (manufactured by Microtec Nition) under ice-cooling. The collagen emulsion thus prepared was intracutaneously administered at 10 sites in the back of each rat at a dose of 0.1 mL/site, to perform initial sensitization of the rat. Seven days after initial sensitization, 0.12 mL of the same collagen emulsion was intracutaneously administered to the tail head of each rat, to perform booster sensitization.
Drug administration was conducted from the day after the initial sensitization through 18 days thereafter. Drug A and Drugs B-1, B-2 and B-4 were all orally administered two times a day, in the morning (9:00 to 11:00 AM) and in the evening (15:30 to 17:30 PM). Drug B-3 was orally administered once a day, around noon (11:30 AM to 13:30 PM).
14 Days and 18 days after the initial sensitization, respectively, the hindlimbs volume was measured again for each rat, and the difference between each of the values and the Pre value was determined to calculate the edema volume for both hindlimbs. The sum of the edema volumes for both hindlimbs measured 14 days and 18 days after the initial sensitization was calculated as the Edema Index, and this was taken as an index for the effect of the drug.

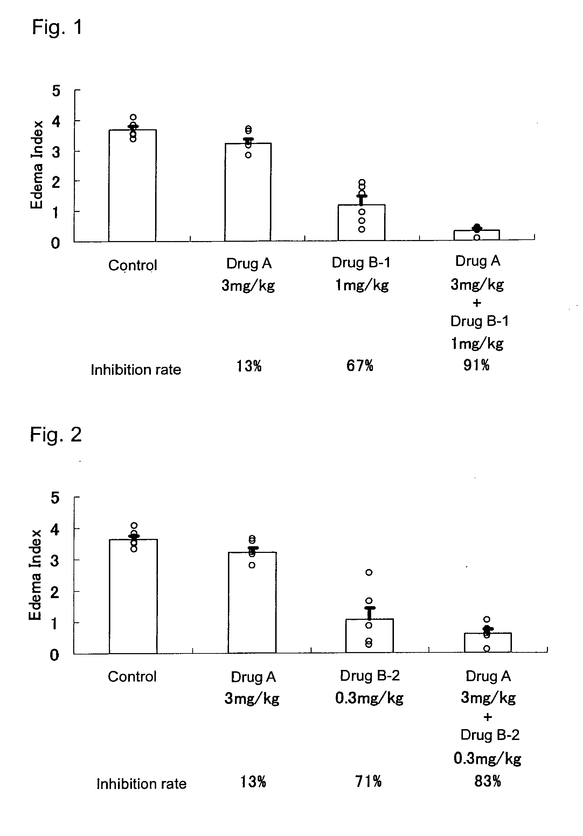

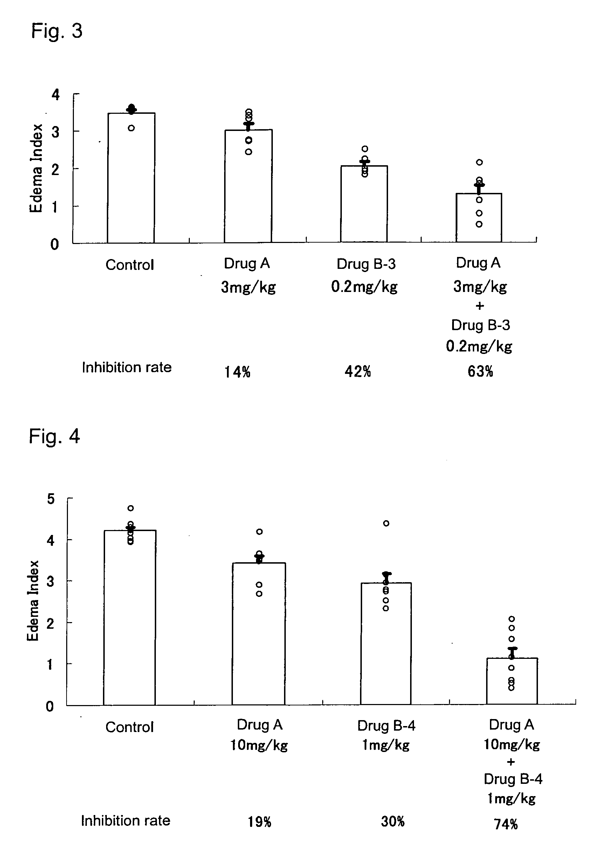

Tables 1 to 4 and Figs. 1 to 4 present the Edema Indices for the group received sole administration of Drug A at a dose of 3 or 10 mg/kg; the group received sole administration of Drug B-1 at a dose of 1 mg/kg; the group received sole administration of Drug B-2 at a dose of 0.3 mg/kg; the group received sole administration of Drug B-3 at a dose of 0.2 mg/kg; the group received sole administration of Drug B-4 at a dose of 1 mg/kg; and the group received combined administrations of two drugs. The Edema Index is represented by an average value ± standard error for 8 rats in each group. Also, the decrease rate is represented by the formula: ((X-Y)/X) × 100 (wherein X: average value of Edema Index for the control group, Y: average value of Edema Index for each group).

**[Table 1]**

| Test drug | Edema Index | Decrease rate (%) | Relative index |
|---|---|---|---|
| Control group | 3.66 ± 0.11 | | |
| Group received sole administration of Drug A at 3 mg/kg | 3.20 ± 0.15 | 13 | 0.87 |
| Group received sole administration of Drug B-1 at 1 mg/kg | 1.20 ± 0.26 | 67 | 0.33 |
| Group received combined administration of Drug A and Drug B-1 | 0.33 ± 0.05 | 91 | 0.09 |

Remark 1) Product of relative indices for the groups received sole administration: 0.87 × 0.33 = 0.29
Remark 2) The edema volume of both hindlimbs represents the average value ± standard error of 6 rats.

The group received combined administration of Drug A and Drug B-1 had a strong effect of decreasing the Edema Index, and the relative index of the Edema Index was smaller than the product of the relative indices of the groups received sole administration of the respective drugs. Thus, a clear synergistic effect of combined administration was recognized.

**[Table 2]**

| Testing drug | Edema Index | Decrease rate (%) | Relative index |
|---|---|---|---|
| Control group | 3.66 ± 0.11 | | |
| Group received sole administration of Drug A at 3 mg/kg | 3.20 ± 0.15 | 13 | 0.87 |
| Group received sole administration of Drug B-2 at 0.3 mg/kg | 1.08 ± 0.35 | 71 | 0.29 |
| Group received combined administration of Drug A and Drug B-2 | 0.62 ± 0.13 | 83 | 0.17 |

Remark 1) Product of relative indices for the groups received sole administration: 0.87 × 0.29 = 0.25
Remark 2) The edema volume of both hindlimbs represents the average value ± standard error of 6 rats.

The group received combined administration of Drug A and Drug B-2 had a strong effect of decreasing the Edema Index, and the relative index of the Edema Index was smaller than the product of the relative indices of the groups received sole administration of the respective drugs. Thus, a clear synergistic effect of combined administration was recognized.

**[Table 3]**

| Testing drug | Edema Index | Decrease rate (%) | Relative index |
|---|---|---|---|
| Control group | 3.48 ± 0.08 | | |
| Group received sole administration of Drug A at 3 mg/kg | 3.00 ± 0.18 | 14 | 0.86 |
| Group received sole administration of Drug B-3 at 0.2 mg/kg | 2.04 ± 0.10 | 42 | 0.58 |
| Group received combined administration of Drug A and Drug B-3 | 1.28 ± 0.25 | 63 | 0.37 |

Remark 1) Product of relative indices for the groups received sole administration: 0.86 × 0.58 = 0.50
Remark 2) The edema volume of both hindlimbs represents the average value ± standard error of 6 rats.

The group received combined administration of Drug A and Drug B-3 had a strong effect of decreasing the Edema Index, and the relative index of the Edema Index was smaller than the product of the relative indices of the groups received sole administration of the respective drugs. Thus, a clear synergistic effect of combined administration was recognized. On the other hand, the group received sole administration of Drug A at 3 mg/kg and the group received sole administration of Drug B at 0.2 mg/kg did not exhibit strong edema suppressing effects, and in both cases, the Edema Index did not decrease to 50% or less of the Edema Index of the control group.

**[Table 4]**

| Testing drug | Edema Index | Decrease rate (%) | Relative index |
|---|---|---|---|
| Control group | 4.20 ± 0.09 | | |
| Group received sole administration of Drug A at 10 mg/kg | 2.93 ± 0.22 | 19 | 0.81 |
| Group received sole administration of Drug B-4 at 1 mg/kg | 3.41 ± 0.16 | 30 | 0.70 |
| Group received combined administration of Drug A and Drug B-4 | 1.11 ± 0.23 | 74 | 0.26 |

Remark 1) Product of relative indices for the groups received sole administration: 0.81 × 0.70 = 0.57
Remark 2) The edema volume of both hindlimbs represents the average value ± standard error of 8 rats.

The group received combined administration of Drug A and Drug B-4 strongly decreased the Edema Index. An F-test for interaction was performed using a statistical means for directly evaluating the synergistic effect (two-way analysis of variance), and a significant synergistic effect (reciprocating) was acknowledged (p = 0.0083), and a clear synergistic effect due to the combined use was confirmed. On the other hand, the group received sole administration of Drug A and the group received sole administration of Drug B did not exhibit strong edema suppressing effects, and in both cases, the Edema Index did not decrease to 50% or less of the Edema Index of the control group.

### EXAMPLE 2

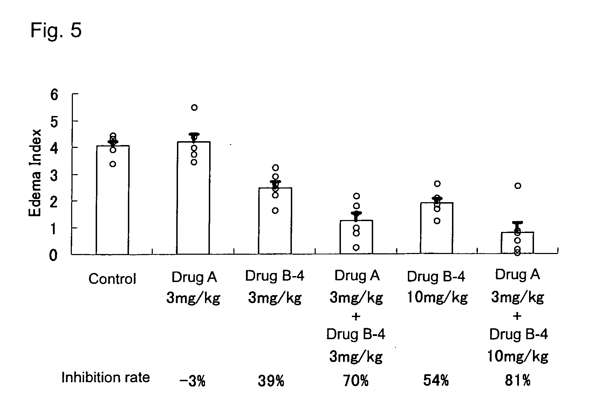

The same procedure as in Example 1 was followed, except that the doses of Drug A and Drug B-4 were changed from 1 mg/kg to 3 or 10 mg/kg, and 6 rats were used, and the Edema Index was measured. The results are presented in Table 5 and Fig. 5.

**[Table 5]**

| Testing drug | Edema Index | Decrease rate (%) | Relative index |
|---|---|---|---|
| Control group | 4.04 ± 0.16 | | |
| Group received sole administration of Drug A at 3 mg/kg | 4.18 ± 0.29 | -3 | 1.03 |
| Group received sole administration of Drug B-4 at 3 mg/kg | 2.46 ± 0.22 | 39 | 0.61 |
| Group received combined administration of Drug A and Drug B-4 (3 mg/kg) | 1.21 ± 0.29 | 70 | 0.30 |
| Group received sole administration of Drug B-4 at 10 mg/kg | 1.86 ± 0.19 | 54 | 0.46 |
| Group received combined administration of Drug A and Drug B-4 (10 mg/kg) | 0.76 ± 0.37 | 81 | 0.19 |

Remark 1) Product of relative indices for the groups received sole administration (Drug A and Drug B-4 at 3 mg/kg) : 1.03 x 0.61 = 0.63
Remark 2) Product of relative indices for the groups received sole administration (Drug A and Drug B-4 at 10 mg/kg): 1.03 x 0.46 = 0.47
Remark 3) The edema volume of both hindlimbs represents the average value ± standard error of 6 rats.

The group for combined administration of Drug A and Drug B-4 had a strong effect of decreasing the Edema Index. A significant synergistic effect (reciprocating) was recognized (p = 0.0243) using an F-test for interaction in the two-way analysis of variance, and a clear synergistic effect due to the combined use was confirmed. On the other hand, the groups received sole administration of Drug B-4 at increased doses of 3 mg/kg and 10 mg/kg did not exhibit strong edema suppressing effects, and the Edema Indices decreased only to 39% and 54%, respectively, of the Edema Index of the control group.

## Claims

1. A preventive and/or therapeutic agent for rheumatoid arthritis, comprising
2-benzyl-5-(4-chlorophenyl)-6-[4-(methylthio)phenyl]-2H-pyr idazin-3-one and a COX2 inhibitor.

2. The preventive and/or therapeutic agent for rheumatoid arthritis according to claim 1,
wherein the COX2 inhibitor is a carboxylic acid-based COX2 inhibitor, an enolic acid-based COX2 inhibitor or a tricyclic-based COX2 inhibitor.

3. The preventive and/or therapeutic agent for rheumatoid arthritis according to claim 1,
wherein the COX2 inhibitor is an inhibitor selected from diclofenac or a salt thereof, ketoprofen or a salt thereof, meloxicam and celecoxib.

4. The preventive and/or therapeutic agent for rheumatoid arthritis according to claim 1,
wherein the rheumatoid arthritis is accompanied with inflammation in joints.

5. The preventive and/or therapeutic agent for rheumatoid arthritis according to claim 1,
wherein the formulation is a preparation for oral administration.

6. A method for treating rheumatoid arthritis, comprising administering effective amounts of
2-benzyl-5-(4-chlorophenyl)-6-[4-(methylthio)phenyl]-2H-pyr idazin-3-one and a COX2 inhibitor.

7. The method according to claim 6,
wherein the COX2 inhibitor is a carboxylic acid-based COX2 inhibitor, an enolic acid-based COX2 inhibitor or a tricyclic-based COX2 inhibitor.

8. The method according to claim 6,
wherein the COX2 inhibitor is an inhibitor selected from diclofenac or a salt thereof, ketoprofen or a salt thereof, meloxicam and celecoxib.

9. The method according to claim 6,
wherein the rheumatoid arthritis is accompanied with inflammation in joints.

10. The method according to claim 6,
wherein the means of administration is oral administration.

11. Use of
2-benzyl-5-(4-chlorophenyl)-6-[4-(methylthio)phenyl]-2H-pyr idazin-3-one and a COX2 inhibitor for the production of a preventive and/or therapeutic agent for rheumatoid arthritis.

12. The use according to claim 11,
wherein the COX2 inhibitor is a-carboxylic acid-based COX2 inhibitor, an enolic acid-based COX2 inhibitor or a tricyclic-based COX2 inhibitor.

13. The use according to claim 11,
wherein the COX2 inhibitor is an inhibitor selected from diclofenac or a salt thereof, ketoprofen or a salt thereof, meloxicam and celecoxib.

14. The use according to claim 11,
wherein the rheumatoid arthritis is accompanied with inflammation in joints.

15. The use according to claim 11,
wherein the formulation is a preparation for oral administration.
